# EUROPEAN PATENT APPLICATION

(11) **EP 4 063 825 A1**
(43) Date of publication of application: **28.09.2022**
(21) Application number: 20891062.0
(22) Date of filing: 30.09.2020
(51) Int. Cl.: G01N 15/05, G01N 1/14, G01N 33/569

(54) **SAMPLE ANALYZER AND SAMPLE ANALYSIS METHOD**

(30) Priority: 18.11.2019 WO PCT/CN2019/119181
(71) Applicant: Shenzhen Mindray Bio-Medical Electronics Co., Ltd., Shenzhen, Guangdong 518057 (CN)
(72) Inventor: LIU, Bin, Shenzhen, Guangdong 518057 (CN); XUN, Wenpeng, Shenzhen, Guangdong 518057 (CN); LI, Chaoyang, Shenzhen, Guangdong 518057 (CN); YI, Qiushi, Shenzhen, Guangdong 518057 (CN); YE, Yi, Shenzhen, Guangdong 518057 (CN)
(74) Representative: KIPA AB
(86) International application number: PCT/CN2020/119436
(87) International publication number: WO 2021/098396

(57) **Abstract**

A sample analyzer and a sample analysis method. A sampling and distribution module (30) collects a blood sample and distributes a first portion of the blood sample to an erythrocyte sedimentation rate measurement module (10) and distributes a second portion of the blood sample to a blood routine test module (20). The sampling and distribution module (30) comprises a sampling needle (31), a power supply pipeline (32) and a sampling power apparatus (33). The erythrocyte sedimentation rate measurement module (10) comprises an erythrocyte sedimentation rate measurement pipeline (11) , an erythrocyte sedimentation rate optical measurement apparatus (12) and an erythrocyte sedimentation rate power apparatus (14), wherein the erythrocyte sedimentation rate measurement pipeline (11) is connected to the end of the sampling needle (31) that is away from a needle head (311). The sampling and distribution module (30) first distributes the second portion of the blood sample to a blood routine reaction cell (21), and then distributes the first portion of the blood sample to the erythrocyte sedimentation rate measurement pipeline (11), wherein the first portion of the blood sample and the second portion of the blood sample are different portions of the same blood sample. Successively distributing completely different portions of the same blood sample to the blood routine test module (20) and the erythrocyte sedimentation rate measurement module (10) can reduce the contamination of a blood section for a blood routine test and improve the accuracy of the blood routine test.

## Description

### TECHNICAL FIELD

The present disclosure relates to the technical field of medical devices, and in particular to a sample analyzer and a sample analysis method.

### BACKGROUND

Erythrocytes in blood in a body are in a dispersed and suspended state due to flowing of the blood and repulsion of negative charges on the surface of erythrocytes. Moreover, when the blood in vitro is left to stand still, the erythrocytes may settle due to gravity. In a pathological state, types and content of proteins in plasma are changed, which changes the balance of charges in the blood, where the negative charges on the surface of erythrocytes are reduced, causing the erythrocytes to be in a rouleaux form and settle more quickly. Therefore, measuring the sedimentation rate of the erythrocytes in one hour, i.e., the Erythrocyte Sedimentation Rate (ESR), assists in disease assessment.

Although the sensitivity and specificity of the ESR in diagnosis are not high, the ESR is still considered as a reliable and indirect acute phase inflammatory response factor, and has a wide clinical application in a long term. For example, the ESR is widely applied to treatment and monitoring of a course of disease such as infectious diseases, acute and chronic inflammation, rheumatism, connective tissue diseases, cancer and Hodgkin's disease.

At present, some methods for predicting the ESR by measuring an erythrocyte aggregation index are known, and these methods indicate that the erythrocyte aggregation index is well correlated with an ESR parameter. In order to measure the erythrocyte aggregation index, the erythrocytes need to be disaggregated, such that the erythrocytes present an independently dispersed state, then transmittance changes in a process from aggregation to disaggregation of the independently dispersed erythrocytes are measured, and thus the aggregation index or ESR is obtained.

Patent CN1864060B issued to the Alifax corporation discloses a method for integrating an ESR module and a cell counting module in series in a blood analyzer. Because the ESR module and the cell counting module are disposed in series on one blood sample loop, a sample acquired by a sampling module flows along the blood sample loop during measurement, the sample is first subjected to an ESR test in the ESR module and then subjected to a blood routine test in the cell counting module. However, in this solution, the blood sample necessarily needs to first flow through the ESR module, and then through a blood routine module, such that the blood sample to be subjected to the blood routine test flows through major areas with diluent, which dilutes and contaminates the blood sample, causing the blood routine test inaccurate.

In addition, in order to disaggregate the erythrocytes before measurement of the ESR, the Alifax corporation also discloses an apparatus for disaggregating erythrocytes by using a peristaltic pump. The peristaltic pump pumps the blood into capillary tubes. When the blood flows in the capillary tubes and the erythrocytes are subjected to a shearing force, an aggregation state of the erythrocytes is broken. After the erythrocytes are dispersed uniformly, the peristaltic pump stops immediately, and the erythrocytes aggregate again. The ESR is measured by measuring a transmittance curve in the aggregation process.

However, in the disaggregation process using the peristaltic pump, in order to enable the erythrocytes to reach a uniform state, the blood needs to flow through a relatively long capillary tube, causing the blood to be diluted by a diluent in the capillary tube, and the measurement precision of the ESR is reduced. Moreover, if the measurement precision is improved in this case, the blood usage amount for the ESR needs to be greatly increased. Because the blood viscosity is high, the blood produces an apparent residual loss when the blood flows in the capillary tubes, and the residual loss of the blood is in direct proportion to the length of the capillary tube through which the blood flows, causing a cleaning time for a pipeline, a cleaning fluid consumption amount and a contamination rate to increase, and also causing the cost of reagents to increase.

### SUMMARY

Therefore, the purpose of the present disclosure is to provide a sample analyzer and a corresponding sample analysis method, capable of reducing contamination on blood segments for blood routine test in the case that a blood routine test and an Erythrocyte Sedimentation Rate (ESR) test are integrated, so as to improve accuracy of the blood routine test; and moreover, an ESR test module is integrated in an existing blood analyzer while a complexity degree of the instrument is not increased and less changes are made as much as possible.

To achieve the purpose of the present disclosure, a first aspect of the present disclosure provides a sample analyzer, including an ESR test module, a blood routine test module, a sampling and distribution module, and a control module.

The sampling and distribution module is configured to acquire a blood sample and distribute a first portion of the blood sample to the ESR test module and a second portion of the blood sample to the blood routine test module. The sampling and distribution module includes a sampling needle, a power supply pipeline, and a sampling power apparatus. The power supply pipeline is configured to connect an end of the sampling needle away from the needle head to the sampling power apparatus, and the sampling power apparatus is configured to drive, through a power pipeline, the sampling needle to suction or discharge the blood sample;

the ESR test module includes an ESR test pipeline, an optical ESR test apparatus, and an ESR power apparatus which is connected to a first end of the ESR test pipeline. The ESR test pipeline is configured to provide a location for test of the distributed first portion of the blood sample. The optical ESR test apparatus is configured to irradiate the first portion of the blood sample distributed to the ESR test pipeline and measure a degree of light absorption or scattering of the first portion of the blood sample to measure an ESR of the blood sample. The ESR power apparatus is configured to suction the first portion of the blood sample to the ESR test pipeline, where a second end of the ESR test pipeline is connected to the end of the sampling needle away from the needle head.

The blood routine test module includes a blood routine reaction pool and a blood routine test apparatus. The blood routine reaction pool is configured to provide a location for mixing the distributed second portion of the blood sample with a reagent, and the blood routine test apparatus is configured to measure at least one blood routine parameter of a blood sample under test obtained by mixing the second portion of the blood sample with the reagent in the blood routine reaction pool.

The control module is connected to the ESR test module, the blood routine test module, and the sampling and distribution module, and configured to: first control the sampling power apparatus to drive the sampling needle to distribute, through the needle head, the second portion of the blood sample to the blood routine reaction pool, and then control the ESR power apparatus to suction the first portion of the blood sample from the end of the sampling needle away from the needle head to the ESR test pipeline, where the first portion of the blood sample and the second portion of the blood sample are different portions of the same blood sample.

A second aspect of the present disclosure provides a sample analysis method, including the following steps.

In a sampling and distribution step, a sampling power apparatus drives, through a power supply pipeline, a sampling needle to suction a blood sample in a sample container, and then distributes a second portion of the blood sample to a blood routine test module.

In an ESR test step, after the blood routine test module completes the blood sample distribution, an ESR power apparatus of the ESR test module suctions a first portion of the blood sample from the sampling needle to an ESR test pipeline of the ESR test module connected to the sampling needle, and an optical ESR test apparatus of the ESR test module irradiates the first portion of the blood sample and measures a degree of light absorption or scattering of the first portion of the blood sample to measure an ESR of the blood sample, where the first portion of the blood sample and the second portion of the blood sample are different portions of the same blood sample.

In a blood routine test step, the blood routine test module, measures at least one blood routine parameter of the distributed second portion of the blood sample.

In the sample analyzer and the sample analysis method according to the present disclosure, the sampling and distribution module first distributes a portion of a blood sample to the blood routine test module and then distributes another completely different portion of the blood sample to the ESR test module. On one hand, contamination on blood segments for blood routine test is reduced, and accuracy of blood routine test is improved. On the other hand, the ESR test module and the blood routine test module perform tests in parallel, and the sample test speed is greatly increased. In addition, by directly connecting the ESR test pipeline to the end of the sampling needle opposite to the needle head, the ESR test module is simply integrated to an existing blood analyzer, and no major structural changes are required.

### BRIEF DESCRIPTION OF THE DRAWINGS

The foregoing and/or additional advantages and features of the present disclosure will become apparent and comprehensible in the description of embodiments made with reference to the following accompanying drawings, where:
FIG. 1 is a schematic structural diagram of a first embodiment of a sample analyzer according to a first aspect of the present disclosure.
FIG. 2 is a schematic structural diagram of a second embodiment of a sample analyzer according to a first aspect of the present disclosure.
FIG. 3 is a schematic structural diagram of a third embodiment of a sample analyzer according to a first aspect of the present disclosure.
FIG. 4 is a schematic structural diagram of a fourth embodiment of a sample analyzer according to a first aspect of the present disclosure.
FIG. 5 is a test sequence diagram of the sample analyzer illustrated in FIG. 4.
FIG. 6 is a schematic structural diagram of a liquid path of a sample analyzer according to a first aspect of the present disclosure.
FIG. 7 is a schematic structural diagram of a fifth embodiment of a sample analyzer according to a first aspect of the present disclosure.
FIG. 8 is a test sequence diagram of the sample analyzer illustrated in FIG. 7.
FIG. 9 is a schematic structural diagram of a sixth embodiment of a sample analyzer according to a first aspect of the present disclosure.
FIG. 10 is a test sequence diagram of the sample analyzer illustrated in FIG. 9.
FIG. 11 is a schematic structural diagram of a seventh embodiment of a sample analyzer according to a first aspect of the present disclosure.
FIG. 12 is an absorbance curve graph of an ESR test process of a sample analyzer according to a first aspect of the present disclosure.
FIG. 13 and FIG. 14 are different test sequence diagrams of a sample analyzer according to a first aspect of the present disclosure.
FIG. 15 to FIG. 17 are block diagrams of different embodiments of a sample analyzer according to a second aspect of the present disclosure.

### DETAILED DESCRIPTION

To make the objectives, technical solutions, and advantages of the present disclosure clearer, the following clearly and completely describes the technical solutions in the embodiments of the present disclosure with reference to the accompanying drawings in the embodiments of the present disclosure. Apparently, the described embodiments are merely some rather than all of the embodiments of the present disclosure. All other embodiments obtained by those skilled in the art based on the embodiments of the present disclosure without creative efforts fall within the scope of protection of the present disclosure.

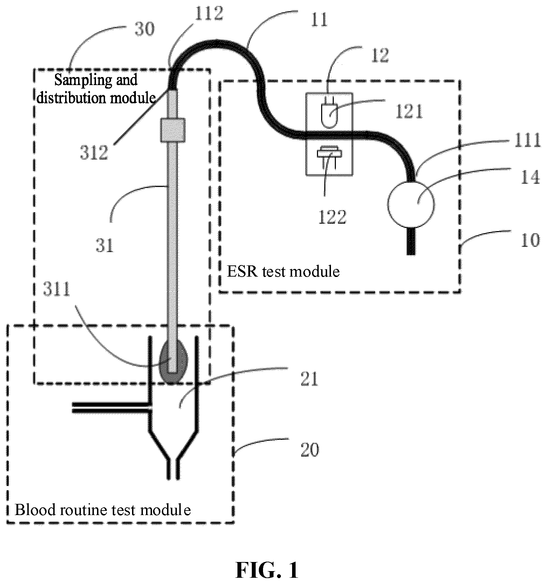

With reference to FIG. 1, a first aspect of the present disclosure provides a sample analyzer, including an ESR test module 10, a blood routine test module 20, and a sampling and distribution module 30.

The sampling and distribution module 30 acquires a blood sample and distributes a first portion of the blood sample (i.e., a first blood sample portion) to an ESR test pipeline 11 of the ESR test module 10 and a second portion of the blood sample (i.e., a second blood sample portion) to the blood routine test module 20. In this case, the sampling and distribution module may be configured to sequentially distribute the first portion and the second portion of the blood sample, or may be configured to simultaneously distribute the first portion and the second portion of the blood sample. The distribution sequence is not limited.

In some embodiments, the sampling and distribution module 30 distributes the first portion of the blood sample to the ESR test pipeline 11 of the ESR test module 10 and the second portion of the blood sample to a blood routine reaction pool 21 of the blood routine test module 20 in a mode of one acquisition and two distributions or a mode of two acquisitions and two distributions.

Specifically, the mode of one acquisition and two distributions is: the sampling and distribution module 30 acquires the blood sample, and the sampling and distribution module 30 first distributes a portion of the acquired blood sample (i.e., the first blood sample portion) to the ESR test pipeline 11 of the ESR test module 10 and then distributes another portion of the acquired blood sample (i.e., the second blood sample portion) to the blood routine reaction pool 21 of the blood routine test module 20, or the sampling and distribution module 30 first distributes a portion of the acquired blood sample (i.e., the second blood sample portion) to the blood routine reaction pool 21 of the blood routine test module 20 and then distributes another portion of the acquired blood sample (i.e., the first blood sample portion) to the ESR test pipeline 11 of the ESR test module 10.

The mode of two acquisitions and two distributions is: the sampling and distribution module 30 first acquires a portion of the blood sample and distributes the acquired portion of the blood sample to the ESR test pipeline 11 of the ESR test module 10 or the blood routine reaction pool 21 of the blood routine test module 20, and the sampling and distribution module 30 then acquires another portion of the blood sample and distributes the acquired another portion of the blood sample to the blood routine reaction pool 21 of the blood routine test module 20 or the ESR test pipeline 11 of the ESR test module 10.

The ESR test module 10 includes the ESR test pipeline 11 and an optical ESR test apparatus 12. The ESR test pipeline 11 is configured to provide a location for test of the distributed first blood sample portion. The optical ESR test apparatus 12 is configured to irradiate the first blood sample portion distributed to the ESR test pipeline 11 and measure a degree of light absorption or scattering of the first blood sample portion in the ESR test pipeline 11 to measure an ESR of the blood sample. when the ESR test apparatus 12 measures the ESR, the first blood sample portion does not move in the ESR test pipeline 11, i.e., keeping still.

The blood routine test module 20 includes the blood routine reaction pool 21 and a blood routine test apparatus (not shown in FIG. 1). The blood routine reaction pool 21 is configured to provide a location for mixing the distributed second blood sample portion with a reagent, and the blood routine test apparatus is configured to carry out a blood routine test on a blood sample under test obtained by mixing the second blood sample portion with the reagent in the blood routine reaction pool 21.

Those skilled in the art can understand that the blood routine test apparatus may be at least one of an optical test unit, an impedance test unit, or a hemoglobin test unit. Accordingly, the blood routine reaction pool can include at least one of an optical reaction pool, an impedance test reaction pool, or a hemoglobin test reaction pool. When carrying out a blood routine test on the blood sample, the blood routine test module 20 adds the blood sample and a corresponding reagent (e.g., a diluent and/or a hemolytic agent and/or a stain) into the blood routine reaction pool 21, and the blood routine test apparatus performs measurement on the blood sample in the blood routine reaction pool 21 to obtain at least one blood routine parameter. The blood routine parameter(s) may include at least one or a combination of multiple of white blood cell (WBC) five-classification result, WBC count and morphological parameters, functional measurements of hemoglobin (HGB), and red blood cell (RBC) and blood platelet (PLT) count and morphological parameters. In an actual blood routine test process, blood routine test items are added or reduced as needed, which is not limited herein.

The sample analyzer according to embodiments of the present disclosure is provided with the ESR test module 10 and the blood routine test module 20. The ESR test module 10 measures the ESR of the blood through the ESR test pipeline 11 and the optical ESR test apparatus 12. The ESR test module 20 measures the blood routine parameter(s). Therefore, the sample analyzer according to the present disclosure can test both the ESR and the blood routine. In practical clinical applications, one device can implement two blood test items, has various functions, and is convenient to use.

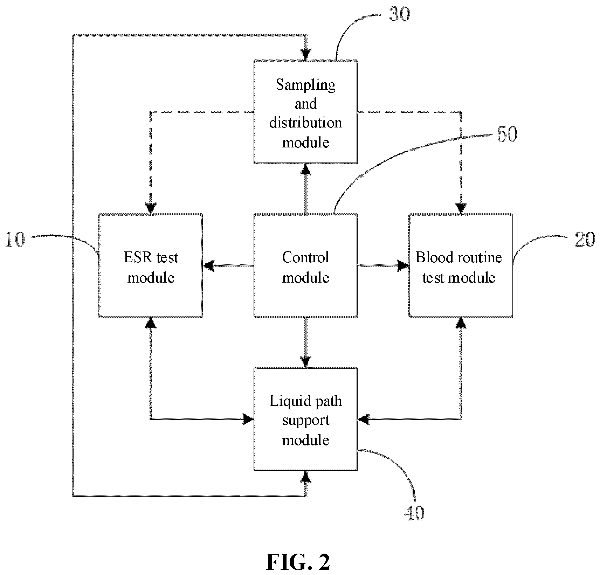

In some embodiments, with reference to FIG. 2, the sample analyzer further includes a liquid path support module 40, configured to provide liquid path support for the sampling and distribution module 30, the ESR test module 10, and the blood routine test module 20. Specifically, the liquid path support module 40 provides the liquid path support by providing liquid for the sampling and distribution module 30, the ESR test module 10, and the blood routine test module 20. For example, the liquid path support module 40 provides respective cleaning liquids for the sampling and distribution module 30, the ESR test module 10, and the blood routine test module 20 for respectively cleaning a sampling needle 31, the ESR test pipeline 11, and the blood routine reaction pool 21, avoiding contamination on the blood sample under test and inaccuracy of a test result. For example, reagent addition, reaction and blending, measurement operations, cleaning and maintenance and the like of the ESR test module and the blood routine test module are all completed under assistance of the liquid path support module.

In addition, the sample analyzer further includes a control module 50. The control module 50 is connected to the sampling and distribution module 30, the ESR test module 10, the blood routine test module 20, and the liquid path support module 40 respectively. The control module 50 is configured to control operations of the sampling and distribution module 30, the ESR test module 10, the blood routine test module 20, and the liquid path support module 40 respectively, so as to enable the sampling and distribution module 30, the ESR test module 10, the blood routine test module 20, and the liquid path support module 40 to cooperate and complete the ESR test and the blood routine test.

In this case, the control module 50 further includes a processor and a memory. The processor is a central processing unit (CPU), a graphical processing unit (GPU), or other chips having the calculation capability. The memory is installed with computer programs executable by the processor such as an operating system and applications, as well as data required for executing the computer programs.

As shown in FIG. 1, the ESR test module 10 further includes a first power apparatus (also referred to as an ESR power apparatus) 14 connected to the ESR test pipeline 11, specifically, a first end 111 of the ESR test pipeline 11. The first power apparatus 14 is configured to drive the first blood sample portion to flow in the ESR test pipeline 11, and enable the first blood sample portion to stop moving and keep still after flowing to a test area 113 in the ESR test pipeline 11, so that the ESR test module 12 measures the ESR of the first blood sample portion. That is, the first power apparatus 14 is not only configured to drive the first blood sample portion to the ESR test pipeline 11, but also configured to ensure that the blood sample in the ESR test pipeline 11 is kept still during measurement of the ESR.

In one specific example, a working process of the ESR test according to the embodiments of the present disclosure is as follows: the first power apparatus 14 drives the first blood sample portion to flow to the ESR test pipeline 11, and after the first blood sample portion flows to a specific position (the test area 113) of the ESR test pipelinell, the first power apparatus 14 immediately stops and instantaneously interrupts the flowing of the first blood sample portion in the ESR test pipeline 11, thereby causing the first blood sample portion to slow down (or stop flowing) at this time, and then aggregation and sedimentation of erythrocytes happen. In the aggregation and sedimentation of the erythrocytes, signal changes are detected by the ESR test apparatus 12, thereby obtaining information for determining the ESR. That is, the control module 50 is configured to control the first power apparatus 14 to drive the first blood sample portion to the test area 113 of the ESR test pipeline 11 and then immediately stop driving of the first power apparatus 14, thereby keeping the first blood sample portion still in the test area 113, so that the optical ESR test apparatus 12 measures the ESR of the first blood sample portion, i.e., measuring the degree of light absorption or scattering of the blood sample in the test area so as to obtain the ESR.

Compared with the existing Westergren method, the sample analyzer according to the embodiments of the present disclosure limits the length of the ESR test pipeline 11, which not only reduces the volume of the entire instrument, but also reduces the blood amount required for test and analysis and the amount of the cleaning liquid for cleaning the ESR test pipeline 11, thereby greatly reducing the cost of the sample analyzer integrating the ESR test and the blood routine test.

In some embodiments, after the ESR is tested, the first power apparatus 14 may further drive, the first blood sample portion to flow in the ESR test pipeline 11 to be discharged from the ESR test pipeline 11, for preparing for the next test.

The first power apparatus 14 may be a pump, or a syringe, or other pressure sources for providing power.

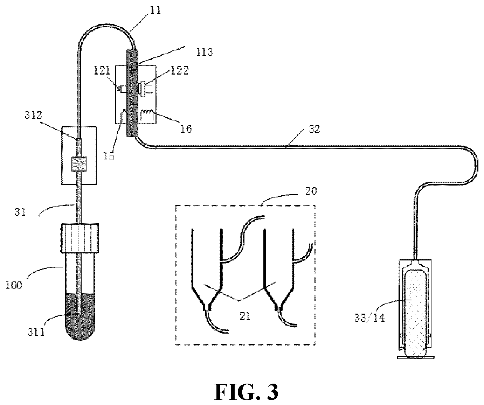

In some embodiments, with reference to FIG. 1 and FIG. 3, the sampling and distribution module 30 may include a sampling needle 31 and a drive apparatus (not shown in the drawings). The drive apparatus is configured to drive the sampling needle 31 to move, so that the sampling needle 31 acquires the blood sample and distributes a portion of the blood sample to the blood routine reaction pool 21 of the blood routine test module 20.

As shown in FIG. 3, the blood sample is generally stored in a test tube 100, and the top of the test tube 100 is provided with a sealing cap. The drive apparatus drives the sampling needle 31 to move to a position above the corresponding test tube 100, and drives the sampling needle 31 to pierce the cap of the test tube by using a needle tip or an end of needle head 311 of the sampling needle 31. Then, the sampling needle 31 extends into the test tube, and suctions the blood sample in the test tube to acquire the blood sample.

In some embodiments, as shown in FIG. 3, the sampling and distribution module 30 may further include a second power apparatus (also referred to as a sampling power apparatus) 33 which is connected, through a power supply pipeline 32, to an end 312 of the sampling needle 31 away from the needle head 311 (that is, the power supply pipeline 32 is configured to connect the end 312 of the sampling needle 31 away from the needle head 311 to the second power apparatus 33). The second power apparatus 33 is configured to drive, through the power supply pipeline 32, the sampling needle 31 to suction or discharge the blood sample through the needle head 311. Furthermore, the second power apparatus 33 is configured to provide a negative pressure for the sampling needle through the power supply pipeline 32 so as to suction the blood sample, and provide a positive pressure to discharge the blood sample. The second power apparatus 33 may be a pump, or a syringe, or other pressure sources for providing power, for example, a positive/negative pressure source. Particularly advantageously, the second power apparatus 33 is implemented as a second syringe.

In some embodiments, as shown in FIG. 1, FIG. 3, and FIG. 4, a second end 112, which is opposite to the first end 111, of the ESR test pipeline 11 is connected to the end 312 of the sampling needle 31 away from the needle head 311. In this case, preferably, an exemplary specific working process of blood distribution of the sample analyzer is as follows: the control module 50 controls the second power apparatus 33 to drive the sampling needle 31 to distribute, through the needle head 311, the second portion of the blood sample to the blood routine reaction pool 21, and then controls the first power apparatus 14 to suction the first portion of the blood sample from the end 312 of the sampling needle 31 away from the needle head 311 to the ESR test pipeline 11, where the first portion of the blood sample and the second portion of the blood sample are different portions of the same blood sample. Therefore, By sequentially distributing completely different portions of the same blood sample to the blood routine test module 20 and the ESR test module 10, contamination on blood segments for blood routine tests is reduced while the blood routine test and the ESR test are integrated, so that accuracy of blood routine tests is improved. In addition, the parallel tests of the blood routine test module 20 and the ESR test module 10 are facilitated.

As described in the background, using a peristaltic pump as a liquid power source of the ESR test module may cause a large increase in the blood usage amount. In addition, because a relatively long test pipeline is needed, a residue amount when a blood sample flows in the test pipeline is increased, which not only causes waste of the blood sample, but also enhances the difficulty in cleaning of the test pipeline and increases the cleaning amount, and thus also increases the possibility of cross contamination. Moreover, the amount of a liquid transmitted by the peristaltic pump may be large or small, the precision is poor, so that the peristaltic pump is suitable for circumstances where low liquid quantitative requirements are provided, and is not suitable for a circumstance having high-precision quantitative (several microliter scale) sample distribution requirements in the field of blood cell measurement. In addition, in an all-in-one machine integrating the blood routine test and the ESR test, using the peristaltic pump as the liquid power source of the ESR test module may cause that two power sources are required in the sampling and distribution module. That is, one is configured to drive a sampling part to suction and discharge a sample, and the other peristaltic pump serves as the liquid power source of the ESR test module. Difficulty is present in deployment of the peristaltic pump and the sampling and distribution module because the peristaltic pump and the sampling and distribution module are difficult to coexist in a blood analyzer. Thus the cost, volume and construction complexity of the all-in-one machine are significantly increased.

Therefore, particularly advantageously, as shown in FIG. 3, the first power apparatus 14 is implemented as a first syringe. The construction solution is not only advantageous to quick and precise EST tests (due to the precise quantitative function of the syringe, the blood sample is precisely suctioned into the test area 113 of the ESR test pipeline 11), but also advantageous to simple and low-cost integration of the ESR test module 10 to an existing blood routine analyzer. Moreover, the volume of the blood routine analyzer is basically not, or slightly increased.

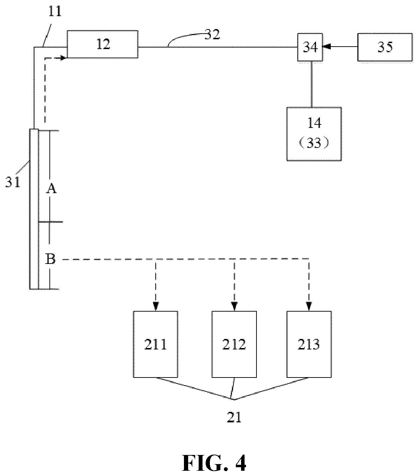

In some embodiments, as shown in FIG. 3 and FIG. 4, the first power apparatus 14 and the second power apparatus 33 may be the same power apparatus. Particularly, the first syringe and the second syringe are the same syringe. As generally, there is a high precision requirement on the second syringe used for quantitative blood distribution for the blood routine test , using the second syringe as the first syringe can not only implement a quick and precise ESR test, but also reduce the cost of sample analysis.

Preferably, in embodiments shown in FIG. 3 and FIG. 4, a part of the power supply pipeline 32 may be the ESR test pipeline 11. For example, the part of the power supply pipeline 32 close to the sampling needle 31 is provided as the ESR test pipeline 11. In this case, the second power apparatus 33 is preferably used as the first power apparatus 14 and is further configured to suction a portion of the blood sample (i.e., the first portion of the blood sample A) in the sampling needle 31 to the ESR test pipeline 11. Moreover, the sampling needle 31 still distributes a portion of the blood sample (i.e., the second portion of the blood sample B) in the sampling needle 31 to the blood routine reaction pool 21 through the needle head 311, to measure the blood routine parameter(s). That is, in this embodiment, the second power apparatus 33, particularly the second syringe, is also used as the first power apparatus 14, particularly the first syringe, and the power supply pipeline 32 of the sampling needle 31 is also used as the ESR test pipeline 11. No additional reaction pool and power apparatus has to be provided. Therefore, the structure is simple, and the cost is reduced. In this case, it can be understood that the second syringe 33 is connected to the power supply pipeline 32 and a diluent storage part 35 respectively through a control valve 34. When the control valve 34 enables the second syringe 33 to be communicated with the diluent storage part 35, the second syringe 33 suctions the diluent from the diluent storage part 35. When the control valve 34 enables the second syringe 33 to be communicated with the power supply pipeline 32, the second syringe 33 provides suction power or discharge power for the sampling needle 31 through the power supply pipeline 32.

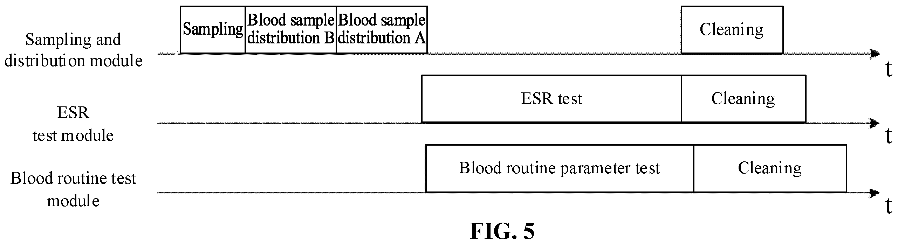

That is, in structure, the ESR test pipeline 11 is directly coupled to a rear pipeline of the sampling needle 31, i.e., the power supply pipeline. Therefore, the ESR test module 10 is simply integrated to an existing blood analyzer without increasing the complexity of the instrument and with less changes as much as possible. As shown in the working sequence diagram in FIG. 5, the second power apparatus 33 (particularly the second syringe) or the first power apparatus 14 (particularly the first syringe) drives the sampling needle 31 to suction the blood sample into the sampling needle 31 until the blood sample flows to the ESR test pipeline 11. Then, the sampling needle 31, for example, first moves to the blood routine reaction pool 21 of the blood routine test module 20 to perform blood distribution (a blood segment B), and after the blood distribution is completed, it is ensured that there is still blood sample (a blood segment A) in the ESR test pipeline 11, or it is ensured that there is still blood sample (the blood segment A) in the sampling needle 31 that can be suctioned to the ESR test pipeline 11. However, the ESR test and the blood routine test are started simultaneously or separately, and it is ensured that during measurement of the ESR, no displacement ofthe blood sample occurs in the ESR test pipeline 11. After the tests are completed, the pipeline is cleaned.

An exemplary specific working process of the blood distribution of the sample analyzer shown in FIG. 4 is as follows: under control of the control module 50, the drive apparatus first drives the sampling needle 31 to move to the test tube 100 loaded with the blood sample.

The second power apparatus 33, i.e., the second syringe here, provides a negative pressure to the sampling needle 31, so that the sampling needle 31 suctions the blood sample in the test tube until the blood sample flows to the ESR test pipeline 11.

The drive apparatus drives the sampling needle 31 to move to the position above the blood routine reaction pool 21, and the second syringe 33 provides a positive pressure to the sampling needle 31 to drop a portion of the blood sample in the sampling needle 31 into the blood routine reaction pool 21 through the needle head 311, to measure the blood routine parameter(s).

Then, the second power apparatus 33 provides a negative pressure to the sampling needle 31, so that the remaining portion of the blood sample in the sampling needle 31 is suctioned to the ESR test pipeline 11 to perform measurement of the ESR. Or, after the sampling needle 31 distributes the blood to the blood routine reaction pool 21, it is ensured that there is still blood sample in the ESR test pipeline 11 for the ESR test module to perform the test, and therefore, there is no need to suction the remaining portion of the blood sample in the sampling needle 31 to the ESR test pipeline 11 through the second power apparatus.

Apparently, in an alternative embodiment, the first power apparatus 14 and the second power apparatus 33 may also be power apparatuses independent from each other. Particularly, the first syringe and the second syringe are syringes independent from each other, and thus different syringes are adopted for different scenarios. The ESR test generally needs a syringe having a flow rate of a 100 uL/S scale (having a high requirement on the flow rate), so as to better implement an erythrocyte disaggregation process which is described in detail later. However, the sampling and blood distribution do not need a large flow rate (having a high requirement on precision), and in this regard, the flow rate of the first syringe (i.e., the ESR power apparatus) may be higher than that of the second syringe (i.e., the sampling power apparatus).

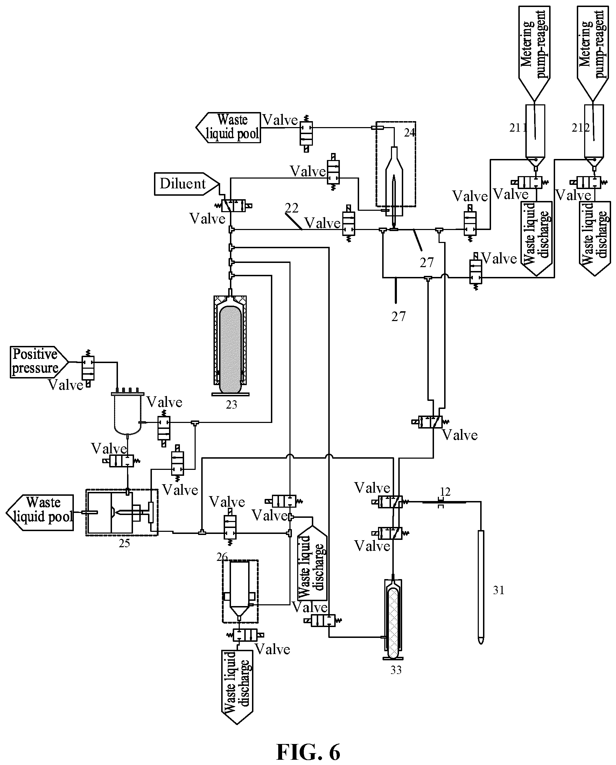

In some embodiments, as shown in FIG. 6, the blood routine test module 20 may include a third syringe 23 which is connected to the blood routine reaction pool 21 through a diluent supply pipeline 22. The third syringe is configured to suction the diluent to the blood routine reaction pool 21 through the diluent supply pipeline 22.

For example, the blood routine reaction pool 21 may include a first optical reaction pool 211 for a leukocyte test and/or a second optical reaction pool 212 for an erythrocyte test (for example, a reticulocyte test). The blood routine test apparatus includes an optical test unit 24. The third syringe 23 is further configured to suction the blood sample under test in the first optical reaction pool 211 and/or the second optical reaction pool 212 to a sample preparation pipeline 27 for an optical test. The second power apparatus 33, which is implemented as the second syringe, is further configured to push the blood sample under test in the sample preparation pipeline 27 for the optical test to the optical test unit 24.

In addition, as shown in FIG. 6, the blood routine reaction pool 21 may further include an impedance measurement reaction pool 213 for an erythrocyte and/or platelet test, and the blood routine test apparatus includes an impedance measurement unit 25. In this case, the third syringe 23 is further configured to suction the blood sample under test in the impedance measurement reaction pool to the sample preparation pipeline for the impedance measurement. The second syringe 33 is further configured to push the blood sample under test in the sample preparation pipeline for the impedance measurement to the impedance measurement unit 25. Alternatively or additionally, the blood routine test apparatus includes a hemoglobin test unit 26. In this case, the third syringe 23 is further configured to suction the blood sample under test to the sample preparation pipeline for the hemoglobin test. The second syringe 33 is further configured to push the blood sample under test in the sample preparation pipeline for the hemoglobin test to the hemoglobin test unit 26.

Preferably, the range of the second syringe 33 is of a microliter scale. More preferably, the range of the second syringe 33 is from 100 uL to 300 uL, for example, 250 uL. Because the range of the second syringe 33 is larger, the quantitative precision of the second syringe 33 is improved on one hand, and a motor having a smaller step pitch is selected for the second syringe 33 as a drive motor on the other hand, for example, for a second syringe 33 with a range of 250 uL, a motor having a step pitch which is half of the step pitch of the motor for a syringe with a range of 100 uL is selected as the drive motor, and thus the requirements on quantitative precision and quantitative volume are both considered. Moreover, the motor operates at a high speed, and the needs of high suctioning and discharge flow rates of the second syringe 33 during the ESR test are satisfied. Preferably, the range of the third syringe 23 is of a milliliter scale, for example, 10 mL.

Those skilled in the art could understand that functions of the second syringe 33 and the third syringe 23 are implemented through a valve control technology.

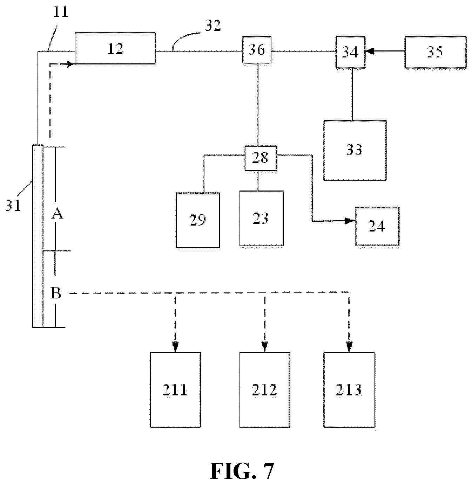

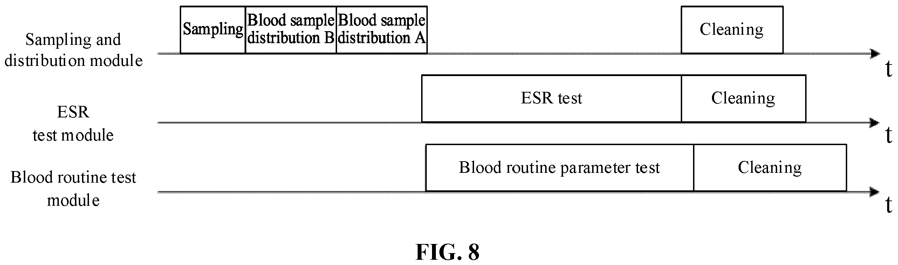

In some embodiments, as shown in FIG. 7, the first power apparatus 14, which is implemented as the first syringe, and the third syringe 23 are the same syringe. In this case, as shown in a working sequence diagram in FIG. 8, the second power apparatus 33, which is implemented as the second syringe, drives the sampling needle 31 to suction the blood sample to the sampling needle 31 until the blood sample flow to the power supply pipeline 32. Then, the sampling needle 31, for example, first moves to the blood routine reaction pool 21 of the blood routine test module 20 to perform blood distribution. Then, by switching between control valves 34, 36 and 28, the third syringe 23 is enabled to drive the remaining blood sample in the sampling needle 31 flow to the ESR test pipeline 11. Then, the ESR test and the blood routine test are started simultaneously or separately, and it is ensured that during measurement of the ESR, no displacement of the blood sample occurs in the ESR test pipeline 11. After the test is completed, pipeline cleaning is performed. In an embodiment shown in FIG. 7, the third syringe 23 is connected to the diluent storage part 29 and the optical test unit 24 through the control valve 28.

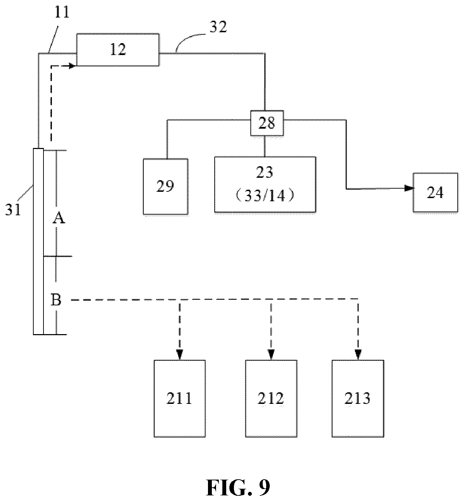

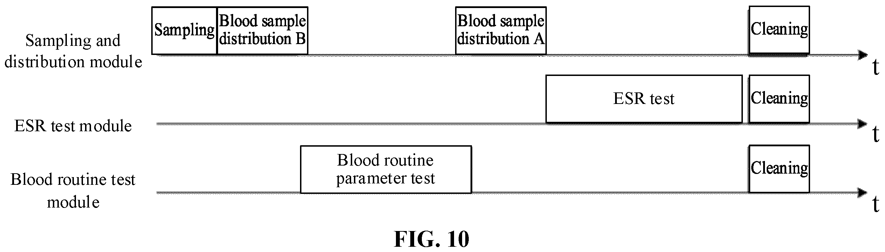

In another embodiments, as shown in FIG. 9, the first power apparatus 14, the second apparatus 33, and the third syringe 23 are the same syringe, so that the cost is further reduced. In this case, as shown in a working sequence diagram in FIG. 10, because there is merely one syringe, the syringe first drives the sampling needle 31 to suction the sample, and then drives the sampling needle 31 to distribute blood (B) to the blood routine test module 20. Then, the blood routine test module 20 first performs the blood routine test, and then after the blood routine test is completed, the merely one syringe drives the remaining blood sample (A) in the sampling needle 31 to flow in the ESR test pipeline 11 to perform measurement of the ESR. After the test is completed, the pipeline is cleaned.

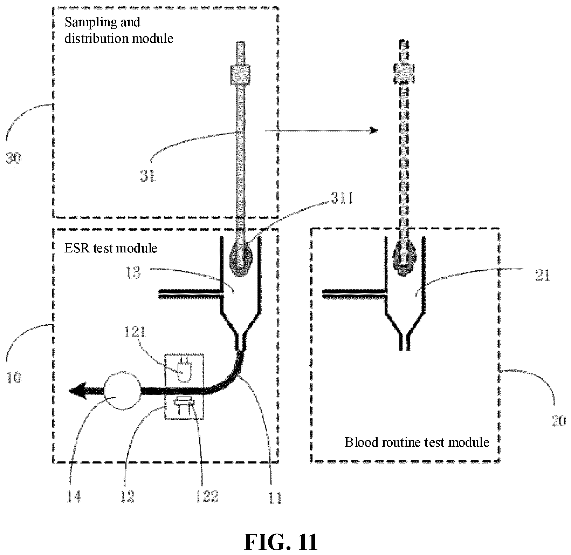

In some alternative embodiments, with reference to FIG. 11, different from the embodiment in which the ESR test pipeline 11 is directly connected to the sampling needle 31, the ESR test module 10 further includes the ESR reaction pool 13 independent from the blood routine reaction pool 21. The ESR reaction pool 13 is connected to one end of the ESR test pipeline 11. The ESR reaction pool 13 is configured to receive the first portion of the blood sample distributed by the sampling needle 31. The first power apparatus 14, particularly the first syringe, is configured to drive the first portion of the blood sample in the ESR reaction pool 13 to flow to the ESR test pipeline 11, and enable the first portion of the blood sample to stop moving and keep still after flowing to the test area of the ESR test pipeline 11, to facilitate the ESR test apparatus 12 to measure the ESR of the first portion of the blood sample.

In an embodiment shown in FIG. 11, an exemplary specific working process of the blood distribution of the sample analyzer according to the present disclosure is as follows, which is performed under control of the control module 50.

The drive apparatus first drives the sampling needle 31 to move to the test tube loaded with the blood sample.

The second power apparatus 33, particularly the second syringe, provides a negative pressure to the sampling needle 31, to facilitate the sampling needle 31 to suction the blood sample in the test tube.

Then, the drive apparatus first drives the sampling needle 31 to move to the position above the ESR reaction pool 13, and the second power apparatus 33 provides a positive pressure to the sampling needle 31 to drop the first portion of the blood sample in the sampling needle 31 into the ESR reaction pool 13. Then, the first power apparatus 14, particularly the first syringe, suctions the blood sample in the ESR reaction pool 13 to the ESR test pipeline 11 to measure the ESR. The drive apparatus then drives the sampling needle 31 to move to the position above the blood routine reaction pool 21, and the second power apparatus 33 provides a positive pressure to the sampling needle 31 to drop the second portion of the blood sample into the sampling needle 31 into the blood routine reaction pool 21, so as to measure the blood routine parameter(s).

Or, the drive apparatus drives the sampling needle 31 to move to the position above the blood routine reaction pool 21, and the second syringe 33 provides a positive pressure to the sampling needle 31 to drop the second portion of the blood sample in the sampling needle 31 into the blood routine reaction pool 21, so as to measure the blood routine parameter(s). The drive apparatus then drives the sampling needle 31 to move to the position above the ESR reaction pool 13, and the second power apparatus 33 provides a positive pressure to the sampling needle 31 to drop the first portion of the blood sample in the sampling needle 31 into the ESR reaction pool 13. Then, the first power apparatus 14 suctions the blood sample in the ESR reaction pool 13 to the ESR test pipeline 11 to measure the ESR.

In this embodiment, after the sampling needle 31 distribute the blood sample to the ESR test module 10 and the blood routine test module 20, the sampling needle 31 may be cleaned, to get prepared for acquisition of a next blood sample.

In order to enable the erythrocytes in the first portion of the blood sample in the ESR test pipeline 11 to be in a dispersed state as much as possible before the ESR test apparatus 12 measures the ESR, to more accurately test the ESR, the first power apparatus 14 reciprocatingly drives the first portion of the blood sample to flow in the ESR test pipeline 11. Specifically, when the first power apparatus 14 is the first syringe, since a movement speed and a movement direction of the syringe can be set flexibly, the disaggregation of the blood sample can be implemented flexibly, and the amount of blood is saved. Pariticularly, when a large-flow high-speed syringe is used, the disaggregation effect can be achieved with a reduced number of reciprocations.

Therefore, in some embodiments, the control module 50 is configured to control, during the test of the ESR test module 10, the first power apparatus 14, particularly the first syringe, of the ESR test module 10, such that:

the first power apparatus 14 drives the first portion of the blood sample to flow back and forth in the ESR test pipeline 11 for a predetermined number of times. It should be noted that the predetermined number of times is a preset numerical value, or may be a value calculated by a processing apparatus such as a controller in a preset calculation manner.

Then, the first power apparatus 14 is enabled to drive the first portion of the blood sample to the test area 113 of the ESR test pipeline 11 and then immediately stop driving, thereby keeping the first portion of the blood sample still in the test area 113 of the ESR test pipeline 11, so as to facilitate the optical ESR test apparatus 12 to measure the ESR of the first portion of the blood sample, i.e., measuring the degree of light absorption or scattering of the blood sample in the test area, so as to obtain the ESR.

Preferably, an elastic deformation coefficient of a pipeline from the sampling needle 31 to the first power apparatus 14, particularly, the first sampling needle, should be designed to ensure that the shearing force in the pipeline satisfies the requirements when the first power apparatus 14 is executing a reciprocating operation for disaggregation. More preferably, the pipeline does not have an adapter, and thus an effect of the adapter on elastic deformation is eliminated.

In a specific embodiment, the control module 50 is configured to enable the first power apparatus 14 to execute the following operations for the predetermined number of times, when controlling the first power apparatus 14, particularly, the first syringe, to drive the first portion of the blood sample to flow back and forth in the ESR test pipeline 11 for the predetermined number of times:

driving the first portion of the blood sample to flow along a first route in the ESR test pipeline 11 at a first speed in a first direction; and

then driving the first portion of the blood sample to flow along a second route, opposite to the first direction, in the ESR test pipeline 11 at a second speed in a second direction.

Preferably, the first speed is identical to the second speed, and the first route is identical to the second route. Therefore, simple control of the first power apparatus 14, particularly, the first syringe, is implemented.

Furthermore, the control module 50 may further control the first power apparatus 14, particularly, the first syringe, according to the blood routine parameter(s) measured by the blood routine test module 20. In this case, the control module 50 is configured to first start the blood routine test module 20 to measure the blood routine parameter(s) of the second portion of the blood sample, and then start the ESR test module 10 to measure an ESR test on the first portion of the blood sample after the blood routine test module 20 completes the measurement of the blood routine parameter(s). Then, the control module 50 is further configured to adjust, during the test of the ESR test module 10, at least one of the first speed, the second speed, the first route, the second route, or the predetermined number of times according to at least one blood routine parameter measured by the blood routine test module 20.

Preferably, the blood routine test module 10 includes an erythrocyte test unit or an impedance test unit configured to measure the hematocrit (HCT), and the at least one blood routine parameter includes the HCT. That is, the erythrocyte test unit of the blood routine test module 10 obtains an HCT value and transmits the HCT value to the control module 50. The control module 50 calculates disaggregation speeds (the first speed and the second speed), routes (the first route and the second route), time and/or the number of reciprocations and the like that are required to be adjusted, and based thereon, controls the first power apparatus 14, particularly, the first syringe to achieve a better disaggregation effect, thereby obtaining higher consistency of ESR results. When the HCT is high, the blood viscosity is increased, and higher disaggregation power or disaggregation time is needed to obtain a good disaggregation performance.

In the case that the first syringe 14 and the second syringe 33 are the same syringe and the ESR test pipeline 11 is a part of the power supply pipeline 32, an exemplary process of disaggregation by repeated suction and discharge is as follows: under control of the control module 50,
the first syringe 14 drives the sampling needle 31 to suction the blood sample.

The sampling needle 31 distributes the blood sample to the blood routine test module 20, for example, the sampling needle 31 is driven by the drive apparatus to move to the position above the blood routine reaction pool, and is driven by the first syringe 14 to distribute blood to the blood routine reaction pool.

The first syringe 14 draws the blood sample to the ESR test area 113.

The first syringe 14 draws the blood sample to move along the first route in the ESR test area at the first speed according to the HCT of the sample.

The first syringe 14 reversely pushes the blood sample to move along the second route at the second speed, which is identical to the first speed, according to the HCT of the sample, the first route being identical to the second route.

The first syringe 14 repeatedly draws and pushes the blood sample for a designed number of times, to homogenize the blood and cause the erythrocytes to be in a dispersed state as much as possible.

The first syringe 14 is stopped immediately. For example, when a step motor is used for driving, the first syringe 14 can be stopped immediately, thereby immediately stopping the movement of the blood sample, and then the erythrocytes in the blood sample aggregate in the test area 113, causing a a transmittance change.

The curve obtained after the first syringe 14 is stopped immediately is integrated and made to correspond to the ESR value, so as to establish association relationship, and finally, the ESR value is reported.

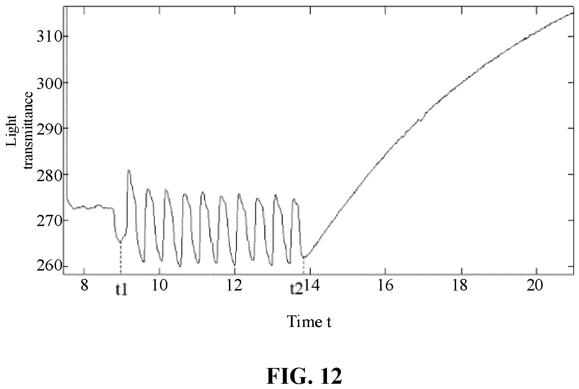

FIG. 12 is a transmittance change curve of the above process, where t1 is a moment when the disaggregation by repeated suction and discharge starts, and t2 is a moment when the first syringe 14 is stopped immediately. In this case, the first syringe 14 drives the blood sample in the ESR test pipeline 11 to move back and forth for five times.

Apparently, the at least one blood routine parameter may also include information indicating whether the blood sample is a chylemia sample.

In some embodiments, a time period in which the ESR test module 10 measures the ESR of the first portion of the blood sample overlaps with a time period in which the blood routine test module 20 measures the blood routine parameter(s) of the second portion of the blood sample.

Different from connecting the ESR test module and the blood routine test module in series and sequentially carrying out a corresponding ESR test and blood routine test in the prior art, the sample analyzer provided by the embodiments of the present disclosure includes the ESR test module 10 and the blood routine test module 20 which can independently carry out tests, rather than waiting for the completion of the test by the previous test module and then entering the test of the next test module. Furthermore, a time period in which the ESR test module 10 measures the ESR of the first portion of the blood sample overlaps with a time period in which the blood routine test module 20 measures the blood routine parameter(s) of the second portion of the blood sample, so that the ESR test module 10 and the blood routine test module 20 can carry out tests in parallel in time, and thus the total time of the two test items is shortened.

Moreover, the sample analyzer provided by the embodiments of the present disclosure is further provided with the sampling and distribution module 30 which is configured to acquire the blood sample. The sampling and distribution module 30 distributes the first portion of the blood sample to the ESR test pipeline 11 of the ESR test module 10 and distributes the second portion of the blood sample to the blood routine test module 20. That is, the sampling and distribution module 30 respectively distributes different portions of the blood sample to the ESR test module 10 and the blood routine test module 20, and thus the ESR test module 10 and the blood routine test module 20 independently carry out the tests in parallel, rather than waiting for the completion of the test by the previous test module and then entering the test of the next test module. The total test time is shortened, and thus, the test efficiency is high. For example, the control module 50 is configured to control the sampling and distribution module 30 to first distributes a portion of the blood sample to the blood routine test module 20 as the second portion of the blood sample, and then distributes the other portion of the blood sample to the ESR test module 10 as the first portion of the blood sample.

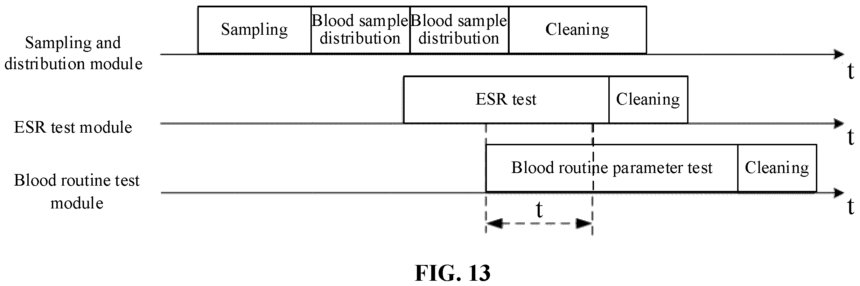

With reference to FIG. 13, FIG. 13 shows a working sequence diagram of the sample analyzer of one example of the present disclosure. The sampling and distribution module 30 first acquires the blood sample and then distributes the first portion of the blood sample to the ESR test module 10. While the sampling and distribution module 30 is distributing the second portion of the blood sample to the blood routine test module 20, the ESR test module 10 starts to measure the ESR of the first portion of the blood sample. After the sampling and distribution module 30 distributes the second portion of the blood sample to the blood routine test module 20, the blood routine test module 20 starts to carry out the blood routine test on the second portion of the blood sample.

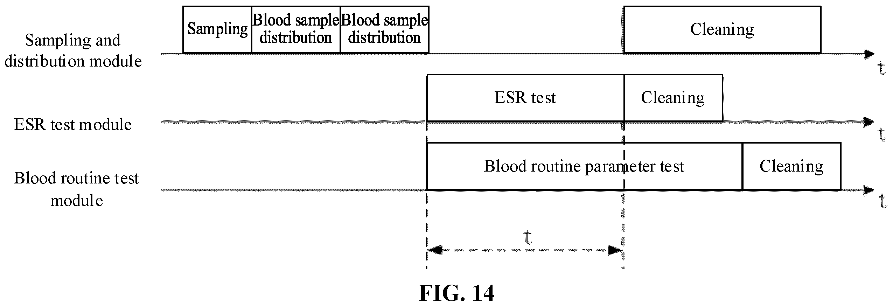

With reference to FIG. 14, FIG. 14 shows another working sequence diagram of the sample analyzer of one example of the present disclosure. Different from FIG. 13, after the sampling and distribution module 30 distributes the first portion of the blood sample to the ESR test module 10 and the second portion of the blood sample to the blood routine test module 20, the ESR test module 10 and the blood routine test module 20 simultaneously start to carry out the ESR test and the blood routine test respectively.

In addition, after the ESR test module 10 and the blood routine test module 20 complete the respective tests, both need to be cleaned for the respective next tests, preventing the residue of the last blood sample under test from affecting a test result of a next blood sample under test. The sampling and distribution module 30 also needs to be cleaned for acquiring the next blood sample under test.

In some embodiments, the first syringe, the second syringe and/or the third syringe include(s) a step motor, a screw and nut assembly, a cylinder body, and a piston disposed in the cylinder body. Liquid is contained in the cylinder body. The screw and nut assembly is connected to the piston. The step motor drives the screw and nut assembly to enable the piston to move in the cylinder body.

In some embodiments, with reference to FIG. 1, FIG. 3, and FIG. 11, the optical ESR test apparatus 12 includes an optical emitter 121 and an optical receiver 122. Each of the optical emitter 121 and the optical receiver 122 is disposed at a respective one of two sides of the test area of the test pipeline 11. The optical emitter 121 is configured to irradiate the first portion of the blood sample. The optical receiver 122 is configured to measure a change amount of the light emitted by the optical emitter 121 after irradiating the first portion of the blood sample (for example, receiving light transmitted and/or scatterred by the first portion of the blood sample), and measure the degree of light absorption or scattering of the first portion of the blood sample by measuring the amount of the received optical signals.

When the test of the ESR test module 10 is started, the first power apparatus 14 drives the first portion of the blood sample to flow into the test pipeline 11, enables the first portion of the blood sample to stop moving and keep still after flowing to the test area. The optical emitter 121 irradiates the first portion of the blood sample in the test area. The optical receiver 122 measures the degree of scattering or transmittance of the light emitted by the optical emitter 121 after irradiating the first portion of the blood sample in the test area, i.e., testing the ESR by measuring the amount of light received by the optical receiver 122.

Because scattering or transmittance of the light irradiating the blood sample may change in a process in which the erythrocytes in the blood sample aggregate (in a rouleaux form), the degree of light scattering or absorption of the first portion of the blood sample is measured by measuring the amount of received transmitted or scattered light after irradiating the blood sample, thereby testing the ESR.

There may be one or more optical receivers 122, which is not limited here.

In one embodiment of the present disclosure, the test pipeline 11 is made of a soft tube, and the test area of the test pipeline 11 is made of a light transmitting material. Therefore, the test pipeline 11 can be disposed freely and flexibly, for example, being disposed vertically, horizontally or inclinedly, or may be disposed to be bent. It is not limited here.

In one embodiment of the present disclosure, the test pipeline 11 is a capillary tube.

In one embodiment of the present disclosure, the ESR test pipeline 11 is made of Fluorinated ethylenepropylene (FEP). When ESR test pipeline 11 is a part of the power supply pipeline 32, the power supply pipeline is made of FEP.

Because the ESR test module 10 provided in the sample analyzer according to the present disclosure measures the ESR by measuring the degree of light scattering or absorption in the aggregation process of erythrocytes in the blood sample (i.e., implementing the ESR test by measuring the aggregation speed of the erythrocytes), compared with a test mode of waiting for natural sedimentation of the erythrocytes due to gravity (the Westergren method), it has a higher test speed, the ESR test can be completed in shorter time (for example, within 20 seconds), and the amount of blood consumption is less, only about 100 microliters (uL). In addition, with respect to the Westergren method, a hard test pipe necessarily needs to be disposed in a straight mode and disposed vertically or slightly inclinedly, and thus the volume of the instrument is prone to being too large. However, the angle of the test pipeline 11 according to the embodiments of the present disclosure is not limited, and the test pipeline 11 can be flexibly adjusted according to the arrangement of other structures inside the sample analyzer, and thus the overall volume of the sample analyzer is reduced, and the space occupied by the sample analyzer is small.

Furthermore, in some embodiments, as shown in FIG. 2, the ESR test module 10 further includes a temperature sensor 15 and a heater 16 that are communicationally connected to the control module 50 and disposed in the vicinity of the test area 113 of the ESR test pipeline 11. The temperature sensor 15 is configured to measure a temperature value of the blood sample in the ESR test pipeline 11 and transmit the temperature value to the control module 50. The control module 50 is configured to control, when the temperature value is lower than a predetermined temperature, the heater 16 to heat the blood sample in the ESR test pipeline 11.

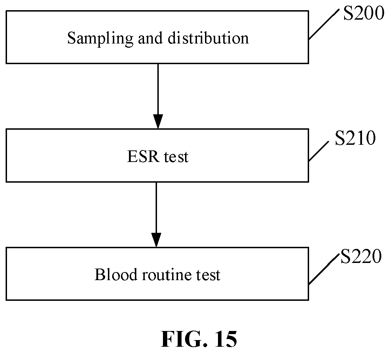

A second aspect of the present disclosure further provides a sample analysis method. As shown in FIG. 15, the sample analysis method further includes the following steps.

In a sampling and distribution step S200, a sampling and distribution module 30 acquires a blood sample in a sample container 100 and distributes a first portion of the blood sample to an ESR test module 10 and a second portion of the blood sample to a blood routine test module 20;

In an ESR test step S210, the ESR test module 10 irradiates the first portion of the blood sample and measures a degree of light absorption or scattering of the first portion of the blood sample to measure an ESR of the first portion of the blood sample; and

In a blood routine test step S220, the blood routine test module 20 measures at least one blood routine parameter of the second portion of the blood sample.

In some embodiments, the sample analysis method may be as follows.

In the sampling and distribution step S200, a sampling power apparatus 33 (also referred to as a second power apparatus) drives, through a power supply pipeline 32, a sampling needle 21 to suction a blood sample in a sample container 100, and then distributes a second portion of the blood sample to a blood routine test module 20.

In the ESR test step S210, after the blood routine test module 20 completes the blood sample distribution, an ESR power apparatus 14 (also referred to as a first power apparatus) of the ESR test module 10 suctions a first portion of the blood sample from the sampling needle 31 to an ESR test pipeline 11 of the ESR test module 10 connected to the sampling needle 31, and an optical ESR test apparatus 12 of the ESR test module 10 irradiates the first portion of the blood sample and measures a degree of light absorption or scattering of the first portion of the blood sample to measure an ESR of the blood sample, where the first portion of the blood sample and the second portion of the blood sample are different portions of the same blood sample.

In the blood routine test step S220, the blood routine test module 20 measures the blood routine parameter(s) of the distributed second portion of the blood sample.

Preferably, in the ESR test step S210, the ESR test module 10 is configured such that the first power apparatus 14 of the first syringe suctions the first portion of the blood sample into the ESR test pipeline 11 of the ESR test module 10.

In some embodiments, in the ESR test step S210, the ESR power apparatus drives the first portion of the blood sample to the test area 113 of the ESR test pipeline 11 and then immediately stops driving, thereby keeping the first portion of the blood sample still in the test area 113, so that the optical ESR test apparatus 12 measures the erythrocyte aggregation rate of the first portion of the blood sample, i.e., measuring the degree of light absorption or scattering of the blood sample in the test area so as to obtain the ESR.

In some embodiments, the sampling and distribution step S200 includes the following.

A drive apparatus of the sampling and distribution module 30 drives the sampling needle 311 of the sampling and distribution module 30 to move to the sample container 100.

The second power apparatus 33 of the sampling and distribution module 30, particularly a second syringe, drives, through the power supply pipeline 32, the sampling needle 311 to suction the blood sample in the sample container 100.

The drive apparatus drives the sampling needle 31 to move the blood routine test module 20.

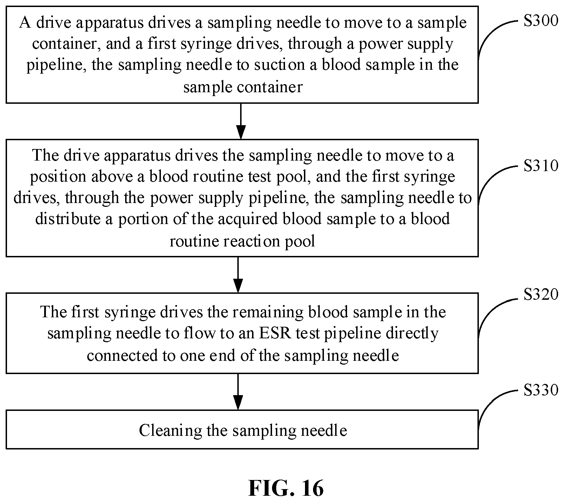

The second power apparatus 33 drives, through the power supply pipeline 32, the sampling needle 311 to distribute the second portion of the blood sample to the blood routine test module 20. In some embodiments, the first power apparatus 14 and the second power apparatus 33 may be the same syringe. In this case, particularly preferably, the ESR test pipeline 11 is a part of the power supply pipeline 32. Here, as shown in FIG. 16, the sample analysis method according to the present disclosure may include the following.

At step S300, a drive apparatus drives a sampling needle 31 to move to a sample container 100, and a first syringe 14 drives, through a power supply pipeline 32, the sampling needle 31 to suction a blood sample in the sample container 100.

At step S310, the drive apparatus drives the sampling needle 31 to move to a position above a blood routine reaction pool 21, and the first syringe 14 drives, through the power supply pipeline 32, the sampling needle 31 to distribute a portion of the acquired blood sample to the blood routine reaction pool 21, for measuring the blood routine parameter(s).

At step S320, the first syringe 14 drives the remaining blood sample in the sampling needle 31 to flow to an ESR test pipeline 11 directly connected to one end of the sampling needle 311, for testing the ESR.

At step S330, after the measurement of the ESR is completed, the sampling needle 31 is cleaned.

In this embodiment, the steps are implemented in a sequence of S300, S310, S320 and S330.

In some embodiments, in the blood routine test step S220, before the blood routine paremeter(s) is tested, a third syringe 23 suctions a diluent to the blood routine test module 20 through a diluent supply pipeline 22, so as to process the second portion of the blood sample.

In this case, the first power apparatus 14 which is configured as the first syringe, and the third syringe 23 may also be the same syringe.

In some embodiments, after the first portion of the blood sample is suctioned to the ESR test pipeline 11 and before the optical ESR test apparatus 12 carries out the ESR test on the first portion of the blood sample, the ESR test step S210 includes the following.

The first power apparatus 14, particularly, the first syringe, drives the first portion of the blood sample to flow back and forth in the ESR test pipeline 11 for a predetermined number of times.

Then, the first power apparatus 14 drives the first portion of the blood sample to the test area 113 of the ESR test pipeline 11 and then immediately stops driving, thereby keeping the first portion of the blood sample still in the test area 113 of the ESR test pipeline 11, so that the optical ESR test apparatus 12 measures the erythrocyte aggregation rate of the first portion of the blood sample, i.e., measuring the degree of light absorption or scattering of the blood sample in the test area so as to obtain the ESR.

Furthermore, the step that the first power apparatus 14, particularly, the first syringe, drives the first portion of the blood sample to flow back and forth in the ESR test pipeline 11 for the predetermined number of times includes performing the following steps for the predetermined number of times:

The first power apparatus 14 drives the first portion of the blood sample to flow along a first route in the ESR test pipeline 11 at a first speed in a first direction.

Then, the first power apparatus 14 then drives the first portion of the blood sample to flow along a second route in the ESR test pipeline 11 at a second speed in a second direction, opposite to the first direction.

Preferably, the first speed is identical to the second speed, and the first route is identical to the second route.

Furthermore, the ESR test process may be adjusted according to the blood routine parameter(s). In this case, the blood routine test module 20 is first started to measure the blood routine parameter(s), and then the ESR test module 10 is started to measure an ESR after the blood routine test module 20 completes measurement of the blood routine parameter(s). In the process of the ESR test step S210, the step that the first power apparatus 14, particularly, the first syringe, drives the first portion of the blood sample to flow back and forth in the ESR test pipeline 11 for the predetermined number of times includes: adjusting at least one of the first speed, the second speed, the first route, the second route, or the predetermined number of times, according to at least one blood routine parameter measured by the blood routine test module 20. Particularly preferably, the at least one blood routine parameter includes the HCT.

In some embodiments, the time at which the ESR test module 10 starts the ESR test on the first portion of the blood sample is identical to or different from the time at which the blood routine test module 20 starts the blood routine parameter measurement on the second portion of the blood sample.

In some embodiments, the sample analysis method further includes the following.

The ESR test module 10 is first started to measure the ESR of the first portion of the blood sample, and then the blood routine test module 20 is started to measure the blood routine parameter(s) of the second portion of the blood sample.

Or, the blood routine test module 20 is first started to measure the blood routine parameters of the second portion of the blood sample, and then the ESR test module 10 is started to measure the ESR of the first portion of the blood sample.

In some embodiments, the sampling and distribution step S200 includes the following.

The sampling and distribution module 30 acquires the blood sample.

The sampling and distribution module 30 first distributes a portion of the acquired blood sample to the ESR test module 10 and then distributes another portion of the acquired blood sample to the blood routine test module 20.

Or, the sampling and distribution module 30 first distributes a portion of the acquired blood sample to the blood routine test module 20 and then distributes another portion of the acquired blood sample to the ESR test module 10.

In some embodiments, the sampling and distribution step S200 includes the following.

The sampling and distribution module 30 first acquires a portion of the blood sample and distributes the acquired portion of the blood sample to the ESR test module 10 or the blood routine test module 20.

The sampling and distribution module 30 then acquires another portion of the blood sample and distributes the acquired another portion of the blood sample to the blood routine test module 20 or the ESR test module 10.

Preferably, the sampling and distribution module 30 first distributes the blood sample to the blood routine test module 20 and starts the blood routine test, and then the sampling and distribution module 30 distributes the blood sample to the ESR test module 10 and starts measurement of the ESR. Since the speed of measurement of the ESR used in the present disclosure is higher than the speed of the blood routine test, first distributing the blood sample to the blood routine test module 20 and starting the blood routine test can facilitate generation of a total test report as quickly as possible.

In some embodiments, the sampling and distribution step S200 includes the following.

The drive apparatus drives the sampling needle 31 to move to the sample container, so as to acquire the blood sample in the sample container.

The drive apparatus drives the sampling needle 31 to move to the ESR test module and the blood routine test module respectively, so as to distribute the first portion of the blood sample to the ESR test module 10 and the second portion of the blood sample to the blood routine test module 20.

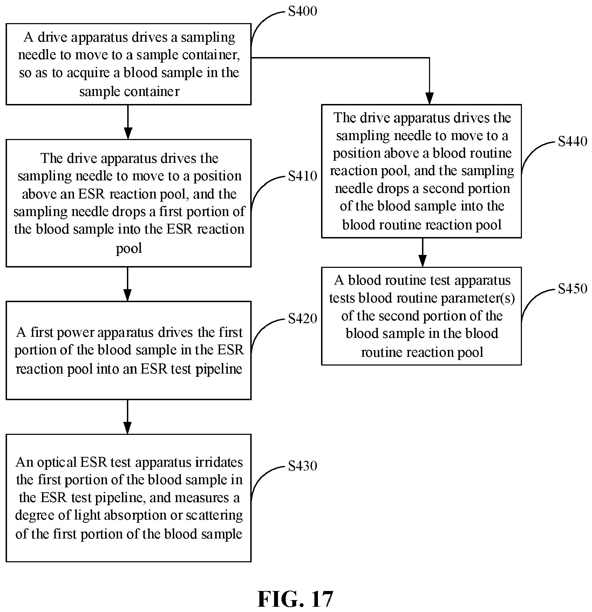

In some embodiments, with reference to FIG. 17, the sample analysis method further includes the following actions.

At S400, the drive apparatus drives the sampling needle 31 to move to the sample container, so as to acquire the blood sample in the sample container.

At S410, the drive apparatus drives the sampling needle 31 to move to a position above the ESR reaction pool 13, and the sampling needle 31 drops the first portion of the blood sample into the ESR reaction pool 13.

At S420, the first power apparatus 14, particularly the first syringe, drives the first portion of the blood sample in the ESR reaction pool 13 to flow to the ESR test pipeline 11, and enables the first portion of the blood sample to stop moving and keep still after flowing to the test area.

At S430, the optical ESR test apparatus 12 irridiates the first portion of the blood sample in the ESR test pipeline 11, and measures the degree of light absorption or scattering of the first portion of the blood sample to measure the ESR.

At S440, the drive apparatus drives the sampling needle 31 to move to a position above the blood routine reaction pool 21, and the sampling needle 31 drops the second portion of the blood sample into the blood routine reaction pool 21.

At S450, the blood routine test apparatus measures the blood routine parameter(s) of the second portion of the blood sample in the blood routine reaction pool 21.

In the embodiments of the present disclosure, S410 is carried out before or after S440. S420 and S430 are carried out before or after S450.

Furthermore, after S410 and S440, the sample analysis method further includes cleaning the sampling needle 31.

In some embodiments, a time period in which the ESR test module 10 measures the ESR of the first portion of the blood sample overlaps with a time period in which the blood routine test module 20 measures the blood routine parameter(s) of the second portion of the blood sample, so that the ESR test module 10 and the blood routine test module 20 can carry out tests concurrently in time to reduce the total time of the two test items. In this case, the sampling and distribution module 30 distributes different portions of the blood sample (i.e., the first portion of the blood sample and the second portion of the blood sample) to the ESR test module 10 and the blood routine test module 20, such that the ESR test module 10 and the blood routine test module 20 independently carry out the tests in parallel, rather than waiting for the completion of the test by the previous test module and then entering the test of the next test module. The total test time is shortened, and thus, the test efficiency is high.

Furthermore, the sample analysis method further includes correcting the ESR according to a result of the blood routine parameter(s). Therefore, a more accurate ESR test result is obtained.

The sample analysis method according to the second aspect of the present disclosure is particularly applied to the sample analyzer according to the first aspect of the present disclosure. For the advantages and more embodiments of the sample analysis method of the present disclosure, reference may be made to the descriptions of the sample analyzer, and the details are not repeated herein.

It should be noted that relational terms such as "first" and "second" are used solely to distinguish one entity or operation from another entity or operation without necessarily requiring or implying any actual such relationship or order between such entities or operations. Moreover, the terms "include", "comprise" or any other variations thereof are intended to cover a non-exclusive inclusion, such that a process, method, object or device including a series of elements not only includes the elements, but also includes other elements not clearly listed, or further includes elements inherent to the process, method, object or device. In the absence of more limitations, an element defined by the statement "including a/an..." does not exclude that the process, method, object or device including the element further has other same elements.

The features mentioned above in the description, the drawings and the claims can be arbitrarily combined with one another as long as they are meaningful within the present disclosure. The features and advantages described for the sample analyzer according to the present disclosure are suitable for the sample analysis method of the present disclosure in a corresponding manner, and vice versa.

The above are merely specific implementations of the present disclosure, so that those skilled in the art can understand or implement the present disclosure. Various modifications to these embodiments are apparent to those skilled in the art, and the generic principles defined herein may be implemented in other embodiments without departing from the spirit or scope of the present disclosure. Therefore, the present disclosure is not intended to be limited to the embodiments herein, but is to be accorded the widest scope consistent with the principles and novel features claimed herein.

## Claims

1. A sample analyzer, comprising an erythrocyte sedimentation rate test module, a blood routine test module, a sampling and distribution module, and a control module, wherein
the sampling and distribution module is configured to acquire a blood sample and distribute a first portion of the blood sample to the ESR test module and a second portion of the blood sample to the blood routine test module, wherein the sampling and distribution module comprises a sampling needle, a power supply pipeline, and a sampling power apparatus, the power supply pipeline is configured to connect an end of the sampling needle away from a needle head to the sampling power apparatus, and the sampling power apparatus is configured to drive, through a power pipeline, the sampling needle to suction or discharge the blood sample;
the ESR test module comprises an ESR test pipeline, an optical ESR test apparatus, and an ESR power apparatus, the ESR test pipeline comprises a first end and a second end opposite to the first end, the first end of the ESR test pipeline is connected to the ESR power apparatus, the ESR test pipeline is configured to provide a location for test of the distributed first portion of the blood sample, the optical ESR test apparatus is configured to irradiate the first portion of the blood sample distributed to the ESR test pipeline and measure a degree of light absorption or scattering of the first portion of the blood sample to measure an ESR of the blood sample, and the ESR power apparatus is configured to suction the first portion of the blood sample to the ESR test pipeline, wherein the second end of the ESR test pipeline is connected to the end of the sampling needle away from the needle head;
the blood routine test module comprises a blood routine reaction pool and a blood routine test apparatus, the blood routine reaction pool is configured to provide a location for mixing the distributed second portion of the blood sample with a reagent, and the blood routine test apparatus is configured to measure a blood routine parameter of a blood sample under test obtained by mixing the second portion of the blood sample with the reagent in the blood routine reaction pool; and
the control module is connected to the ESR test module, the blood routine test module, and the sampling and distribution module, and is configured to: first control the sampling power apparatus to drive the sampling needle to distribute, through the needle head, the second portion of the blood sample to the blood routine reaction pool, and then control the ESR power apparatus to suction the first portion of the blood sample from the end of the sampling needle away from the needle head to the ESR test pipeline, wherein the first portion of the blood sample and the second portion of the blood sample are different portions of the same blood sample.

2. The sample analyzer of claim 1, wherein the ESR test pipeline is a part of the power supply pipeline.

3. The sample analyzer of claim 1 or 2, wherein the sampling power apparatus and the ESR power apparatus are a same syringe.

4. The sample analyzer of claim 1 or 2, wherein the sampling power apparatus and the ESR power apparatus are individual syringes, and a flow rate in the ESR power apparatus is higher than that in the sampling power apparatus.

5. The sample analyzer of any one of claims 1 to 4, wherein the control module is configured to control the ESR power apparatus to drive the first portion of the blood sample to a test area of the ESR test pipeline, and then immediately stop driving the ESR power apparatus, to keep the first portion of the blood sample still in the test area, so as to enable the optical ESR test apparatus to measure the degree of light absorption or scattering of the blood sample in the test area to obtain the ESR.

6. The sample analyzer of any one of claims 1 to 5, wherein the control module is configured to first start the blood routine test module to measure the a blood routine parameter of the second portion of the blood sample, and then start the ESR test module to measure the ESR of the first portion of the blood sample.

7. The sample analyzer of claim 6, wherein the control module is configured to enable a time period in which the ESR test module measures the ESR of the first portion of the blood sample to overlap with a time period in which the blood routine test module measures the a blood routine parameter of the second portion of the blood sample.

8. The sample analyzer of claim 6, wherein the control module is configured to start the ESR test module to measure the ESR of the first portion of the blood sample after the blood routine test module completes measurement of the a blood routine parameter.

9. The sample analyzer of any one of claims 1 to 8, wherein in order to control the ESR test module to measure the ESR of the first portion of the blood sample, the control module is configured to control the ESR power apparatus of the ESR test module to:
enable the ESR power apparatus to drive the first portion of the blood sample to flow back and forth in the ESR test pipeline for a predetermined number of times; and
then enable the ESR power apparatus to drive the first portion of the blood sample to a test area of the ESR test pipeline and then immediately stop driving, to keep the first portion of the blood sample still in the test area, so as to enable the optical ESR test apparatus to measure the degree of light absorption or scattering of the blood sample in the test area to obtain the ESR.

10. The sample analyzer of claim 9, wherein in order to control the ESR power apparatus to drive the first portion of the blood sample to flow back and forth in the ESR test pipeline for the predetermined number of times, the control module is configured to enable the ESR power apparatus to execute the following operations for the predetermined number of times:
driving the first portion of the blood sample to flow along a first route the ESR test pipeline at a first speed in a first direction; and
then driving the first portion of the blood sample to flow along a second route in the ESR test pipeline at a second speed in a second direction, which is opposite to the first direction.

11. The sample analyzer of claim 10, wherein the first speed is identical to the second speed, and the first route is identical to the second route.

12. The sample analyzer of claim 10 or 11, wherein the control module is configured to first start the blood routine test module to measure the a blood routine parameter of the second portion of the blood sample, and then start the ESR test module to measure the ESR of the first portion of the blood sample after the blood routine test module completes measurement of the a blood routine parameter; and
the control module is further configured to adjust at least one of the first speed, the second speed, the first route, the second route, or the predetermined number of times according to at least one of the a blood routine parameter of the blood sample.

13. The sample analyzer of claim 12, wherein the blood routine test module comprises an erythrocyte test unit configured to measure hematocrit, and the at least one of the a blood routine parameter comprises the hematocrit.

14. A method for sample analysis, comprising:
a sampling and distribution step: driving, by a sampling power apparatus, through a power supply pipeline, a sampling needle to suction a blood sample in a sample container, and then distributing, by the sampling power apparatus, a second portion of the blood sample to a blood routine test module;
an ESR test step: after the blood routine test module completes the blood sample distribution, suctioning, by an ESR power apparatus of an ESR test module, a first portion of the blood sample from the sampling needle to an ESR test pipeline of the ESR test module connected to the sampling needle, and irradiating, by an optical ESR test apparatus of the ESR test module, the first portion of the blood sample, and measuring, by the optical ESR test apparatus, a degree of light absorption or scattering of the first portion of the blood sample to measure an ESR of the blood sample, wherein the first portion of the blood sample and the second portion of the blood sample are different portions of the same blood sample; and
a blood routine test step: measuring, by the blood routine test module, a blood routine parameter of the distributed second portion of the blood sample.

15. The sample analysis method of claim 14, wherein in the ESR test step, the ESR power apparatus drives the first portion of the blood sample to a test area of the ESR test pipeline and then immediately stops driving to keep the first portion of the blood sample still in the test area, to enable the optical ESR test apparatus to measure the degree of light absorption or scattering of the blood sample in the test area, so as to obtain the ESR.

16. The sample analysis method of claim 14 or 15, comprising: first starting measurement of the a blood routine parameter of the blood routine test module, and then starting measurement of the ESR of the ESR test module.

17. The sample analysis method of claim 16, wherein a time period in which the ESR test module measures the ESR of the first portion of the blood sample overlaps with a time period in which the blood routine test module measures the a blood routine parameter of the second portion of the blood sample.

18. The sample analysis method of claim 16, wherein the measurement of the ESR of the ESR test module is started after the blood routine test module completes the measurement of the a blood routine parameter.

19. The sample analysis method of any one of claims 14 to 18, wherein in the ESR test step, after the ESR power apparatus suctions the first portion of the blood sample to the ESR test pipeline and before the ESR test module measures the ESR of the first portion of the blood sample, the ESR test step further comprises:
enabling the ESR power apparatus to drive the first portion of the blood sample to flow back and forth in the ESR test pipeline for a predetermined number of times; and
then enabling the ESR power apparatus to drive the first portion of the blood sample to the test area of the ESR test pipeline and then immediately stop driving, to keep the first portion of the blood sample still in the test area of the ESR test pipeline, to enable the optical ESR test apparatus to measure the degree of light absorption or scattering of the blood sample in the test area, so as to obtain the ESR.

20. The sample analysis method of claim 19, wherein the enabling the ESR power apparatus to drive the first portion of the blood sample to flow back and forth in the ESR test pipeline for predetermined number of times comprises:
driving, by the ESR power apparatus, another portion of the blood sample to flow along a first route in the ESR test pipeline at a first speed in a first direction; and
then driving, by the ESR power apparatus, the another portion of the blood sample to flow along a second route in the ESR test pipeline at a second speed in a second direction opposite to the first direction.

21. The sample analysis method of claim 20, wherein the first speed is identical to the second speed, and the first route is identical to the second route.

22. The sample analysis method of claim 20 or 21, comprising first starting measurement of the a blood routine parameter by the blood routine test module, and then starting measurement of the ESR of the ESR test module after the blood routine test module completes the blood routine parameter measurement,
wherein the enabling the ESR power apparatus to drive the first portion of the blood sample to flow back and forth in the ESR test pipeline the predetermined number of times comprises:
adjusting, according to at least one blood routine parameter of the blood sample, at least one of the first speed, the second speed, the first route, the second route, or the predetermined number of times.

23. The sample analysis method of claim 22, wherein the at least one of the a blood routine parameter comprises hematocrit.

24. The sample analysis method of any one of claims 14 to 23, further comprising: correcting the ESR according to a result of the a blood routine parameter.

25. The sample analysis method of any one of claims 14 to 24, wherein the sample analysis method is applied to the sample analyzer of any one of claims 1 to 13.
